# FASCICULE DE BREVET EUROPEEN

(11) **EP 4 408 298 B1**
(45) Date de publication et mention de la délivrance du brevet: **17.06.2026**
(21) Numéro de dépôt: 22782513.0
(22) Date de dépôt: 20.09.2022
(51) Int. Cl.: A61B 10/00, A61F 5/44, A61G 9/00

(54) **DISPOSITIF DE RECUEILLEMENT DE LIQUIDES**
VORRICHTUNG ZUM SAMMELN VON FLÜSSIGKEITEN
DEVICE FOR COLLECTING LIQUIDS

(30) Priorité: 28.09.2021 FR 2110230
(43) Date de publication de la demande: 07.08.2024
(73) Titulaire: Swiss Safe Collect SA, 2000 Neuchâtel (CH)
(72) Inventeur: CAILLETEAU, Benoît, 1807 BLONAY (CH)
(74) Mandataire: Cabinet Beau de Loménie
(86) Numéro de dépôt international: PCT/EP2022/076126
(87) Numéro de publication internationale: WO 2023/052204

(56) Documents cités:
- EP-A1- 1 695 678
- SE-C2- 528 318
- US-A- 5 312 379
- US-A1- 2004 006 321
- US-A1- 2017 079 571
- US-B1- 6 685 684

## Description

### Domaine Technique

Le présent exposé concerne un dispositif de recueillement de liquides, par exemple de liquides biologiques tels que de l'urine ou des vomissures.

### Technique antérieure

On connaît dans l'état de la technique des dispositifs de recueillement de l'urine ou de vomissures par exemple dans le brevet européen n° 3174512 présentant une poche munie d'un clapet de sécurité pour sensiblement empêcher le contenu de l'enveloppe de sortir par l'ouverture.

Ces dispositifs sont par exemple utilisés par des patients alités, par exemple en hôpital, ou des personnes peu mobiles.
US 6685684 B1, SE 528318C2, US 2004/006321 A1, US 5312379 A,
EP 1695678 A1, et US 2017/079571 A1 concernent des exemples de dispositifs de recueillement des urines.

### Exposé de l'invention

La nécessité de recueillir des liquides d'un patient est connue, par exemple pour réduire un risque de contamination par ces liquides, et pour en assurer l'élimination.

L'intérêt de l'analyse des liquides d'un patient pour l'évaluation d'une pathologie ou de l'état de santé du patient est connu, comme base de la biologie médicale.

Couramment, les liquides d'un patient sont transférés entre différents contenants entre leur recueillement, leur analyse et leur élimination, ce qui augmente le risque de contamination liée à une mauvaise manipulation.

Le problème s'est donc posé de concevoir un dispositif permettant d'assurer de manière sûre et fiable les fonctions de recueillement des liquides, et pouvant être facilement éliminé.

Le présent exposé vise à répondre à ce problème.

La présente invention concerne un dispositif de recueillement de liquides tel que défini en revendication 1, le dispositif comprenant :
une poche souple comprenant des parois configurées pour ménager entre elles une ouverture d'introduction de liquides,
un dispositif réactif comprenant au moins un élément réactif à au moins un composant d'un liquide, et un agencement de protection configuré pour protéger le dispositif réactif vis-à-vis d'un contact avec un jet de liquide provenant de l'ouverture d'introduction de liquides.

On entend par liquides tout type de liquides, par exemple des liquides biologiques d'un utilisateur, comme les urines ou les vomissures, ou un mélange de liquides biologiques. On entend également par liquides des liquides non biologiques, par exemple des liquides en phase aqueuse tels que des eaux usées ou tout autre liquide industriel, ou des liquides en phase non aqueuse, comme du pétrole ou des huiles.

Le dispositif de recueillement de liquides peut ainsi avoir une application dans des domaines médicaux ou non médicaux.

On entend par élément réactif à un composant d'un liquide, un dispositif qui réagit de façon caractéristique en réponse à un contact avec un liquide et/ou avec des espèces chimiques contenues dans le liquide.

On entend par poche souple une poche pouvant se déformer lors du remplissage par des liquides. L'utilisation d'une poche souple permet par exemple de réduire le volume du dispositif de recueillement de liquides avant utilisation afin de faciliter son stockage. On comprend toutefois que la poche peut également être une poche rigide, ou semi-rigide.

On entend par souple, un matériau qui peut se déformer sous son propre poids. On entend par semi-rigide un matériau qui se déforme peu sous son propre poids, mais qui peut facilement se déformer à la main. On entend par rigide un matériau qui se déforme peu sous son propre poids et qui se déforme difficilement à la main.

Une telle poche permet de recueillir des liquides tout en protégeant le dispositif réactif d'un contact non souhaité avec les liquides. Le dispositif réactif peut ensuite être mis en contact avec les liquides recueillis de façon à réaliser la réaction entre l'élément réactif et les liquides.

Un tel dispositif permet alors un recueillement de liquides de façon sûre et fiable, tout en réduisant les phases de manipulation nécessaires au contact et à la réaction du liquide avec l'élément réactif.

Le dispositif réactif comprend un dispositif d'analyse de liquides.

Le dispositif d'analyse de liquides est un élément réactif à un composant d'un liquide, au sens où il permet de réaliser une analyse du liquide suite à un contact avec ce liquide. On entend par analyse de liquides la détection de la présence d'au moins un composant dans les liquides et/ou la mesure de la concentration d'au moins un composant dans les liquides. Le composant peut être une espèce chimique d'origine biologique, telle qu'une protéine, ou d'origine non biologique. Le composant peut également être une cellule, un tissu biologique ou une bactérie. Les résultats d'analyse sont lisibles par un utilisateur. La lecture par un utilisateur peut être réalisée visuellement, par exemple par comparaison avec une échelle colorimétrique ou lecture d'un indicateur, ou par lecture d'un signal, par exemple un signal électrique ou numérique.

Avec cette configuration, les liquides peuvent être recueillis dans la poche souple sans entrer en contact avec le dispositif d'analyse de liquides, ce qui permet d'éviter un contact prématuré du dispositif d'analyse de liquides avec les liquides recueillis, un tel contact prématuré risquant de dégrader la qualité des mesures du dispositif d'analyse de liquides voire d'empêcher une mesure. De cette façon, la durée de contact des liquides recueillis avec le dispositif d'analyse de liquides peut être contrôlée, de façon à assurer le bon fonctionnement du dispositif d'analyse de liquides.

En particulier, dans le cas où les liquides recueillis sont les urines d'un patient, la mise en contact contrôlée de l'urine et du dispositif d'analyse de liquides permet d'éviter un contact avec l'urine de début de miction, laquelle peut être inadaptée pour la réalisation de certaines analyses.

Le dispositif réactif comprend un matériau absorbant.

Le matériau absorbant est un élément réactif à un composant d'un liquide, au sens où il permet d'absorber des liquides, par exemple en les retenant dans une phase solide ou dans un gel. Le matériau absorbant peut également floculer les liquides.

Une telle poche permet de recueillir des liquides, puis de les retenir, par exemple dans une phase solide ou dans un gel et ainsi de faciliter leur élimination par des filières de gestion de déchets adaptées. Dans le cas où les liquides sont des urines d'un patient suivant un traitement médical, par exemple une chimiothérapie, l'absorption et l'élimination des urines permet de réduire la pollution des eaux usées par des résidus médicamenteux toxiques.

De plus, la protection par l'agencement de protection permet de recueillir les liquides dans la poche souple sans qu'ils entrent en contact avec le matériau absorbant, ce qui entraînerait une absorption prématurée risquant d'empêcher une analyse des liquides ou de nuire à la fiabilité d'une telle analyse.

L'agencement de protection est configuré pour protéger le dispositif d'analyse de liquides vis-à-vis d'un contact avec le matériau absorbant.

Cette configuration permet d'éviter toute interférence entre les fonctions de recueillement, d'analyse, et d'absorption des liquides, qui peuvent être alors être assurées de manière sûre et fiable par le dispositif.

Les résultats d'analyse de liquides réalisés par le dispositif de la présente invention pourront dans un second temps être soumis à l'expertise d'un professionnel de la santé, qui pourra comparer des valeurs mesurées par le dispositif avec les valeurs de référence, par exemple les valeurs nominales d'un patient en bonne santé afin de réaliser un diagnostic.

Dans certains modes de réalisation, l'agencement de protection est configuré pour autoriser le contact de liquides recueillis dans la poche souple avec le matériau absorbant et avec le dispositif d'analyse de liquides.

Une fois les liquides recueillis, le contact avec le matériau absorbant et avec le dispositif d'analyse de liquides permet respectivement de réaliser les fonctions d'absorption et d'analyse des liquides.

Dans certains modes de réalisation, le dispositif comprend un clapet anti retour configuré pour faire obstacle à une circulation de liquides recueillis dans la poche souple vers l'extérieur de la poche souple.

Cette configuration permet d'empêcher les liquides de s'échapper de la poche souple. En particulier, cette configuration permet d'autoriser des manipulations de la poche afin de mettre en contact les liquides avec le matériau absorbant et avec le dispositif d'analyse de liquides tout en prévenant une fuite accidentelle de liquides hors de la poche souple.

Dans certains modes de réalisation, l'agencement de protection comprend au moins une partie du clapet anti retour.

Le clapet anti retour peut alors avoir une double fonction de protection du dispositif d'analyse de liquides et du matériau absorbant d'un jet de liquide provenant de l'ouverture d'introduction de liquides, et de prévention d'une fuite accidentelle de liquides hors de la poche souple. De cette façon, la structure du dispositif de recueillement de liquides est simplifiée, ce qui réduit davantage les risques de défaillance du dispositif.

Dans certains modes de réalisation, le clapet anti retour comprend un clapet anti retour à feuilles.

Dans certains modes de réalisation, l'agencement de protection comprend une portion de feuilles souples qui délimite une première chambre avec une première paroi de la poche souple et qui délimite une deuxième chambre avec une deuxième paroi de la poche souple.

La première chambre et la deuxième chambre peuvent alors recevoir de manière séparée des liquides recueillis.

Dans certains modes de réalisation, le dispositif d'analyse de liquides est situé dans la première chambre et le matériau absorbant est situé dans la deuxième chambre, le dispositif d'analyse de liquides et le matériau absorbant étant positionnés dans des zones comprises entre l'ouverture d'introduction de liquides et une extrémité de l'agencement de protection opposée à l'ouverture d'introduction de liquides.

Une telle configuration permet de distribuer les liquides recueillis entre une première chambre dans laquelle les liquides recueillis peuvent entrer en contact avec le dispositif d'analyse de liquides, et une deuxième chambre dans laquelle les liquides recueillis peuvent entrer en contact avec le matériau absorbant. De cette façon, les fonctions d'analyse des liquides et d'absorption des liquides peuvent être réalisées sans interférer entre elles.

Dans certains modes de réalisation, le dispositif d'analyse de liquides est fixé à l'une de la première paroi et de la portion de feuilles souples, et le matériau absorbant est fixé à l'une de la deuxième paroi et de la portion de feuilles souples.

Un tel positionnement permet d'assurer le positionnement du dispositif d'analyse de liquides et du matériau absorbant respectivement dans la première chambre et dans la deuxième chambre même en cas de manipulation du dispositif de recueillement par un utilisateur.

Dans certains modes de réalisation, le matériau absorbant est prévu dans une enveloppe soluble ou libre dans la deuxième chambre ou dans la poche.

Par soluble, on comprend soluble dans les liquides considérés. Dans le cas de liquides en phase aqueuse, par exemple des urines ou des vomissures, soluble signifie hydrosoluble.

L'enveloppe soluble permet de maintenir en position le matériau absorbant jusqu'à un contact avec les liquides, suite à quoi l'enveloppe soluble dissoute peut libérer le matériau absorbant pour absorber les liquides résiduels de la poche.

Dans certains modes de réalisation, le matériau absorbant comprend du polyacrylate de sodium. D'autres matériaux absorbants peuvent être prévus en fonction de la nature des liquides à absorber, par exemple parmi des polymères superabsorbants.

Dans certains modes de réalisation, le dispositif d'analyse de liquides comprend une bandelette réactive de test.

Une bandelette réactive de test comprend des zones réactives qui réagissent au contact de liquides et permettent de mesurer de manière qualitative ou semi-quantitative la présence de certains paramètres des liquides. Un professionnel de la santé pourra ensuite comparer des valeurs mesurées par le dispositif avec les valeurs référence, par exemple les valeurs nominales d'un patient en bonne santé, puis réaliser un diagnostic.

Dans certains modes de réalisation, le dispositif d'analyse de liquides comprend un laboratoire sur puce (ou « Lab-on-chip », en langue anglaise). Dans certains modes de réalisation, le laboratoire sur puce comprend un dispositif de transmission de données de mesures, qui peuvent alors être recueillies et consultées par un personnel médical, et qui pourra alors comparer des valeurs mesurées par le dispositif avec les valeurs références d'un patient en bonne santé puis réaliser un diagnostic. Ces résultats de mesures peuvent également être utilisés par un utilisateur autre qu'un personnel médical afin de détecter des taux anormaux de grandeurs d'intérêt, par exemple de taux de créatinine pour des sportifs ou de nitrites et de leucocytes pour des personnes susceptibles aux infections urinaires. Un utilisateur peut alors prendre contact avec un personnel médical apte à réaliser un diagnostic.

L'invention concerne également un procédé tel que défini en revendication 13, le procédé étant un procédé de recueillement de liquides d'un ou par un utilisateur à l'intérieur d'une poche souple comprenant des parois configurées pour ménager entre elles une ouverture d'introduction de liquides, un dispositif réactif comprenant au moins un élément réactif à au moins un composant d'un liquide, le procédé comprenant les étapes suivantes :
recueillement dans la poche souple de liquides d'un ou par un utilisateur à travers l'ouverture d'introduction de liquides ; protection du dispositif réactif vis-à-vis d'un contact avec un jet de liquide provenant de l'ouverture d'introduction de liquides.

Le procédé comprend les étapes suivantes :
protection d'un matériau absorbant du dispositif réactif vis-à-vis d'un contact avec un dispositif d'analyse de liquides du dispositif réactif.

Dans certains modes de réalisation, le procédé comprend les étapes suivantes :
entrave d'une circulation de liquides recueillie dans la poche souple vers l'extérieur de la poche souple ; retournement de la poche souple de manière à baigner le dispositif d'analyse de liquides et le matériau absorbant dans les liquides recueillis dans la poche souple pendant une durée d'exposition prédéterminée.

Par baigner, on comprend immerger totalement ou partiellement. Par exemple, par baigner un dispositif réactif dans les liquides, on comprend mettre en contact au moins l'élément réactif du dispositif réactif avec les liquides.

Dans certains modes de réalisation, le procédé comprend le relevé d'une mesure du dispositif d'analyse de liquides et l'absorption de liquides recueillie dans la poche souple par le matériau absorbant.

### Brève description des dessins

[Fig. 1A] [Fig. 1B] [Fig. 1C] [Fig. 1D] [Fig. 1E] Les figures 1A à 1E représentent une même vue latérale d'un dispositif de recueillement de liquides selon respectivement un premier, un deuxième, un troisième, un quatrième et un cinquième modes de réalisation; le premier, le deuxième, et le cinquième modes de réalisation étant utiles à la compréhension de l'invention mais ne faisant pas partie de l'invention telle que revendiquée.
[Fig. 2A][Fig. 2B] Les figures 2A et 2B sont des vues schématiques en coupe du dispositif de recueillement de liquides du premier et du deuxième mode de réalisation.
[Fig. 2C][Fig. 2D] Les figures 2C et 2D sont des vues schématiques en coupe du dispositif de recueillement de liquides du troisième et du quatrième mode de réalisation.
[Fig. 3A] La figure 3A est une vue schématique en coupe d'un dispositif de recueillement de liquides du premier mode de réalisation en position retournée après recueillement des liquides.
[Fig. 3B] La figure 3B est une vue schématique en coupe d'un dispositif de recueillement de liquides du troisième mode de réalisation en position retournée après recueillement des liquides.
[Fig. 4] La figure 4 est une vue schématique en coupe d'un dispositif de recueillement de liquides après recueillement des liquides, ce dispositif étant utile à la compréhension de l'invention mais ne faisant pas partie de l'invention telle que revendiquée.

### Description des modes de réalisation

La présente invention sera décrite en se référant à des exemples de réalisation spécifiques. Toutefois, il est évident que des modifications et des changements peuvent être effectués sur ces exemples sans sortir de la portée générale de l'invention telle que définie par les revendications. En particulier, des caractéristiques individuelles des différents modes de réalisation illustrés/mentionnés peuvent être combinées dans des modes de réalisation additionnels. Par conséquent, la description et les dessins doivent être considérés dans un sens illustratif plutôt que restrictif.

La présente invention concerne un dispositif de recueillement de liquides 100, et sera décrit dans le cas d'une poche comprenant un clapet de sécurité à feuilles. Une poche comprenant un clapet de sécurité à feuilles est connue de l'homme du métier, par exemple dans le brevet européen n° 3174512, au contenu duquel on pourra se référer lors de la lecture du présent exposé, et ne sera pas décrit en détail.

Dans le présent exposé, le dispositif de recueillement de liquides 100 sera décrit dans une repère orthogonal direct d'axes X, Y, Z, dans lequel, par convention, l'axe Z est vertical. De fait, lorsque des liquides sont recueillis dans le dispositif, celui-ci est normalement positionné de sorte que son ouverture d'introduction soit orientée vers le haut, de sorte que l'axe Z est alors vertical ou en tout cas incliné par rapport à l'horizontale.

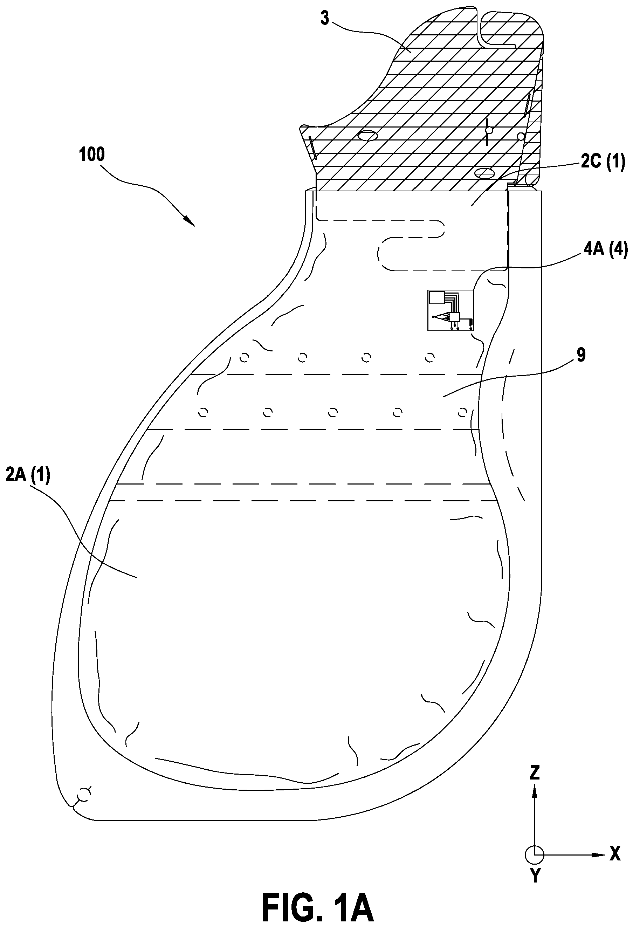

Comme représenté dans la vue selon le plan YZ de la Figure 1A, le dispositif de recueillement de liquides 100 comprend une poche souple 1. La poche souple 1 est délimitée par une première paroi 2A et une deuxième paroi 2B reliées l'une à l'autre sur la quasi-totalité des contours des parois 2A, 2B, par exemple par une soudure. Les parties libres des contours des parois 2A, 2B ménagent un col 2C sur lequel est fixé un manchon 3.

La poche souple 1 peut être réalisée en une matière plastique, par exemple du polyéthylène.

Le dispositif de recueillement de liquides 100 comprend un dispositif réactif comprenant un élément réactif à au moins un composant d'un liquide. Par exemple, l'élément réactif est un dispositif d'analyse de liquides 4 sous forme d'un laboratoire sur puce 4A.

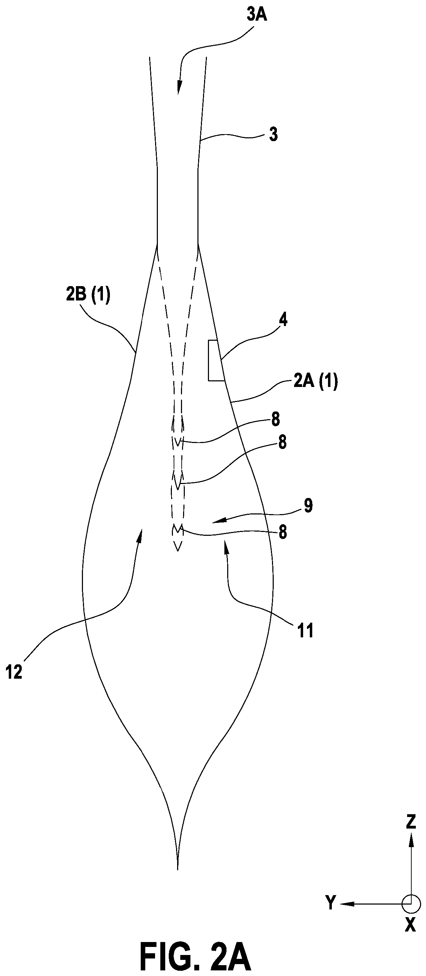

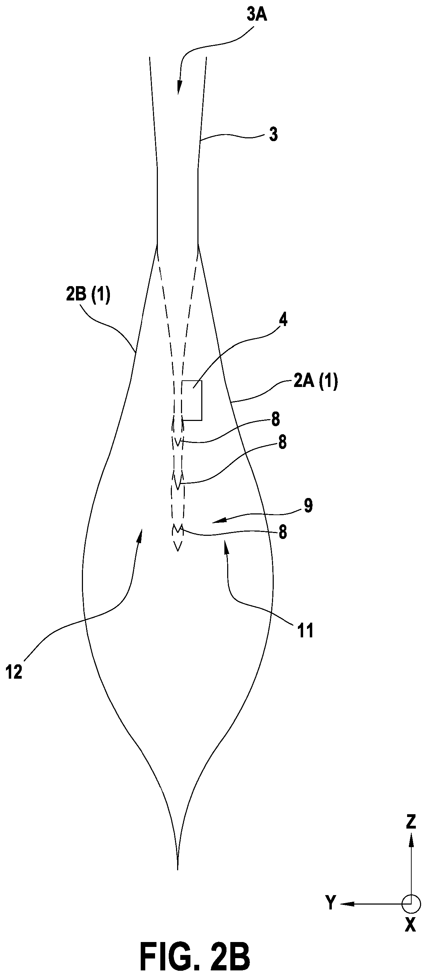

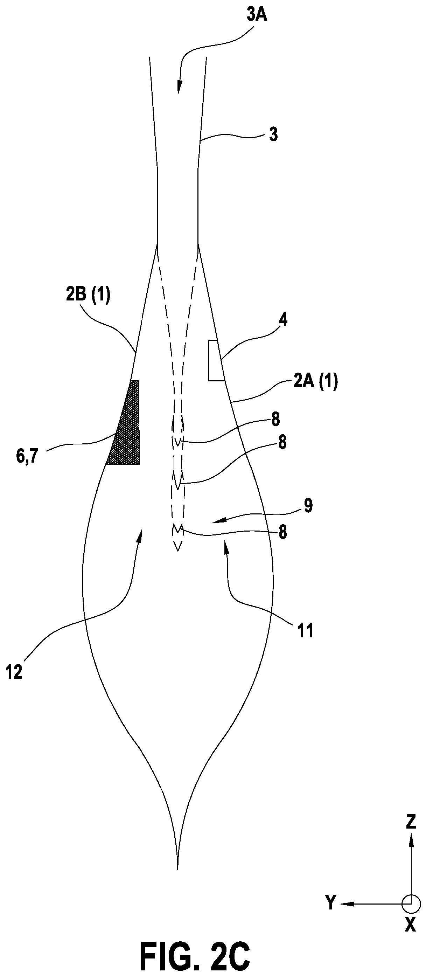

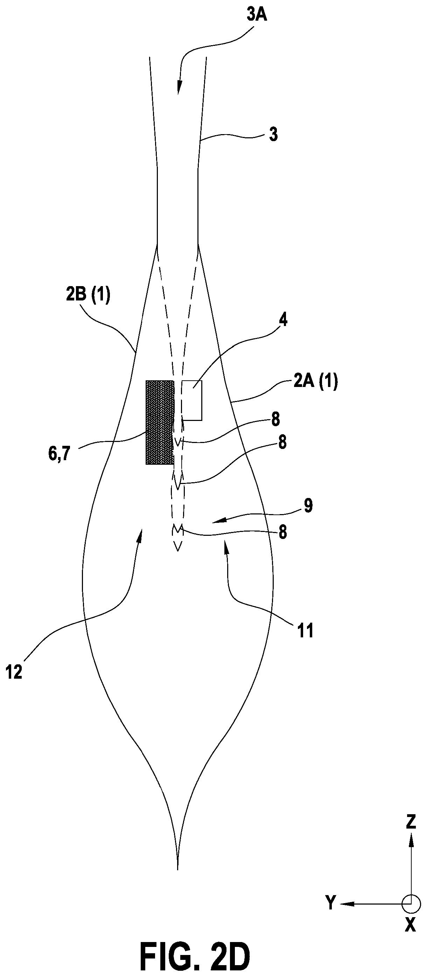

Le dispositif de recueillement de liquides 100 comprend également un matériau absorbant 6 et un clapet anti retour 9, qui seront décrits plus en détail relativement aux figures 2 à 4.

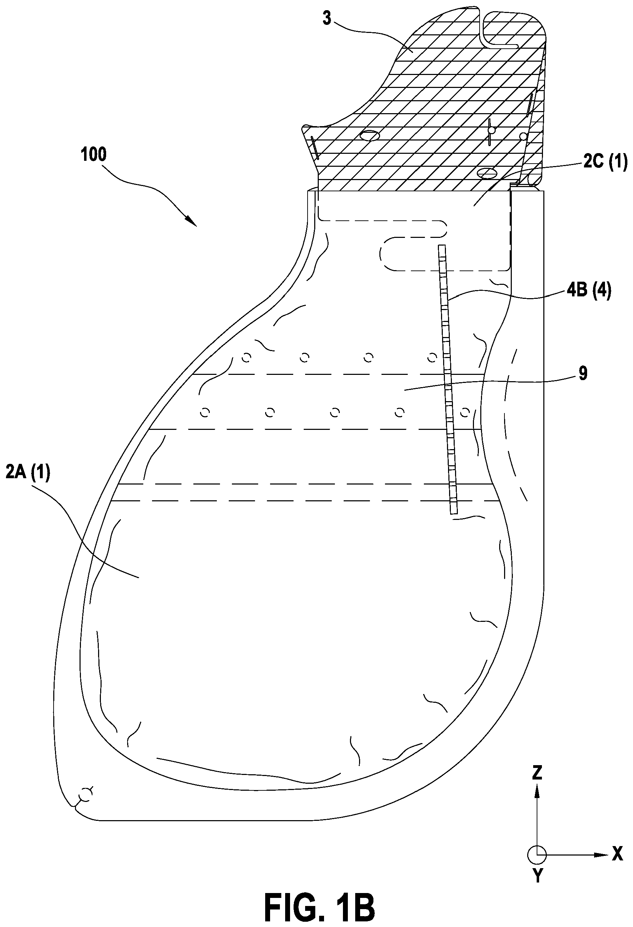

Les modes de réalisations des figures 1A et 1B diffèrent en ce que dans le mode de réalisation de la figure 1B, le dispositif d'analyse de liquides 4 est une bandelette réactive de test 4B. De manière générale, le dispositif d'analyse de liquides 4 peut comprendre une bandelette réactive de test 4B et/ou un laboratoire sur puce 4A.

Une bandelette réactive de test est connue de la personne du métier, et est une bandelette munie de marqueurs réagissant à la présence voire à la concentration de certains marqueurs chimiques ou biologiques, entraînant par exemple l'apparition d'un motif ou un changement de couleur.

Un laboratoire sur puce est connu de l'homme du métier, et est un dispositif de mesure biochimique miniaturisé.

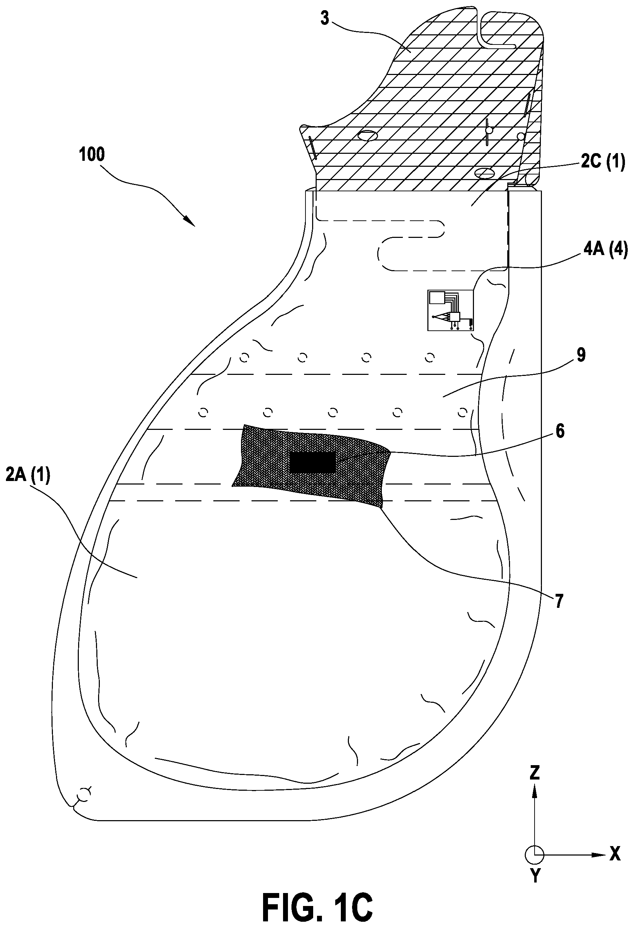

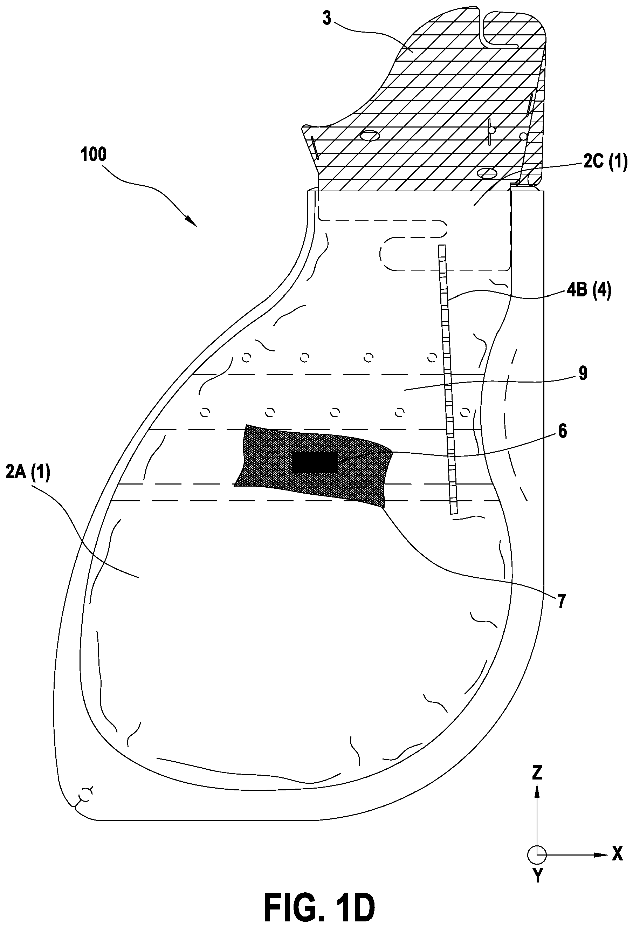

Les modes de réalisation des figures 1C et 1D correspondent respectivement aux modes de réalisation des figures 1A et 1B, dans lesquels le dispositif réactif comprend en outre un élément réactif sous forme d'un matériau absorbant 6.

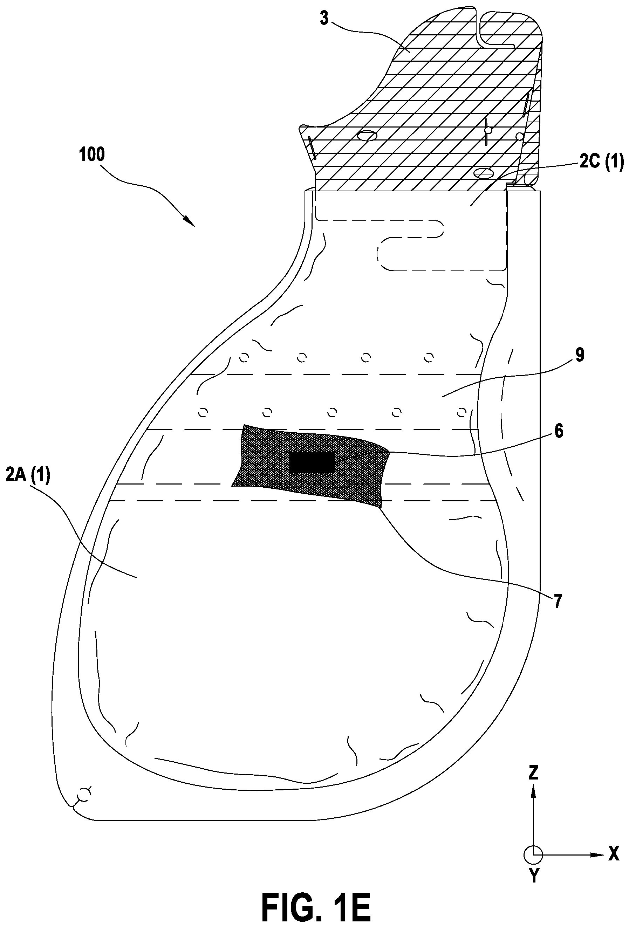

Comme représenté sur la figure 1E, le dispositif réactif peut également comprendre un matériau absorbant 6 et le dispositif d'analyse de liquides 4 être absent.

Le positionnement du dispositif d'analyse de liquides 4 et du matériau absorbant 6 dans la poche souple 1 sera décrit en référence aux figures 2A à 2D.

La figure 2A représente une vue schématique en coupe du dispositif de recueillement et d'analyse de liquides 100 selon le plan YZ, orthogonal au plan XZ de la figure 1A.

Le manchon 3 délimite une ouverture d'introduction de liquides 3A par laquelle des liquides peuvent entrer dans le dispositif de recueillement de liquides 100.

Un agencement de protection 9 est prévu, afin de protéger le dispositif réactif vis-à-vis d'un contact avec un jet de liquide provenant de l'ouverture d'introduction de liquides 3A.

Par exemple, l'agencement de protection 9 protège le dispositif d'analyse de liquides 4 et/ou le matériau absorbant 6 vis-à-vis d'un contact avec un jet de liquide provenant de l'ouverture d'introduction de liquides 3A.

L'agencement de protection 9 forme un passage d'écoulement de liquides dans le prolongement de l'ouverture d'introduction de liquides 3A.

L'agencement de protection 9 est par exemple un clapet anti retour 9.

En particulier, le clapet anti retour 9 est un clapet anti retour 9 à feuilles comprenant des paires de feuilles 8 reliées entre elles par des points de liaisons prévus de manière à autoriser le passage de liquides venant de l'ouverture d'introduction de liquides 3A jusqu'à un intérieur de la poche souple 1, tout en prévenant une circulation de liquides depuis l'intérieur de la poche souple 1 vers l'extérieur de la poche souple 1.

Comme représenté sur les figures 1A et 1E, le clapet anti retour 9 s'étend entre deux extrémités du contour des parois 2A, 2B, de sorte que la première paroi 2A et des feuilles 8 du clapet anti retour 9 délimitent une première chambre 11, et que la deuxième paroi 2B et des feuilles 8 du clapet anti retour 9 délimitent une deuxième chambre 12, représentées sur la figure 2A.

On comprend que la distinction entre la première paroi 2A et la deuxième paroi 2B, et entre la première chambre 11 et la deuxième chambre 12 est arbitraire, et que celles-ci sont interchangeables.

La première chambre 11 et la deuxième chambre 12 sont séparées par les feuilles 8 du clapet anti retour 9, et forment chacune une cavité ouverte qui peuvent communiquer fluidiquement entre elles par-delà une extrémité des feuilles 8 du clapet anti retour 9.

On comprend que l'extension de la première chambre 11 et de la deuxième chambre 12 dépend de l'extension du clapet anti retour 9. En particulier, la première chambre 11 et la deuxième chambre 12 sont ici délimitées par un plan perpendiculaire à la direction d'écoulement de liquides depuis l'ouverture d'introduction de liquides 3A et passant par une extrémité des feuilles 8 du clapet anti retour 9, c'est-à-dire un plan perpendiculaire à la direction Z dans la représentation de la figure 2A. Autrement dit, la première chambre 11 et la deuxième chambre 12 sont comprises entre l'ouverture d'introduction de liquides 3A et une extrémité du clapet anti retour 9 opposée à l'ouverture d'introduction de liquides 3A.

La première chambre 11 et la deuxième chambre 12 correspondent alors à des positions intérieures à la poche souple 1 qui ne peuvent pas recevoir de liquides lors du recueillement d'un jet de liquide depuis l'ouverture d'introduction de liquides lorsque la poche est en position verticale.

Le dispositif d'analyse 4 est prévu dans la première chambre 11. Le dispositif d'analyse 4 est par exemple fixé à la première paroi 2A, comme représenté sur la figure 2A, ou fixé aux feuilles 8 délimitant la première chambre 11, comme représenté dans le mode de réalisation de la figure 2B.

Les modes de réalisation des figures 2C et 2D correspondent respectivement aux modes de réalisation des figures 2A et 2B dans lesquels le dispositif de recueillement de liquides 100 comprend en outre un matériau absorbant 6.

Le matériau absorbant 6 est prévu dans la première chambre 11. Le matériau absorbant 6 est par exemple fixé à la deuxième paroi 2B, comme représenté sur la figure 2C, ou fixé aux feuilles 8 délimitant la deuxième chambre 12, comme représenté sur la figure 2D.

L'agencement de protection 9 est prévu pour protéger le dispositif d'analyse de liquides 4 vis-à-vis d'un contact avec le matériau absorbant 6.

Le matériau absorbant 6 peut être prévu dans une enveloppe soluble 7. L'enveloppe soluble est par exemple une enveloppe hydrosoluble 7, par exemple comprenant des polymères hydrosolubles tels que des polymères de la famille des dextrines et des caséines.

Le fonctionnement de la poche sera décrit en référence aux figures 2A à 4.

Après introduction des liquides par l'ouverture d'introduction de liquides 3A, les liquides s'écoulent et sont recueillis dans la poche souple 1 sans entrer en contact ni avec le dispositif d'analyse de liquides 4, et le cas échéant avec le matériau absorbant 6, protégé(s) par le clapet anti retour 9.

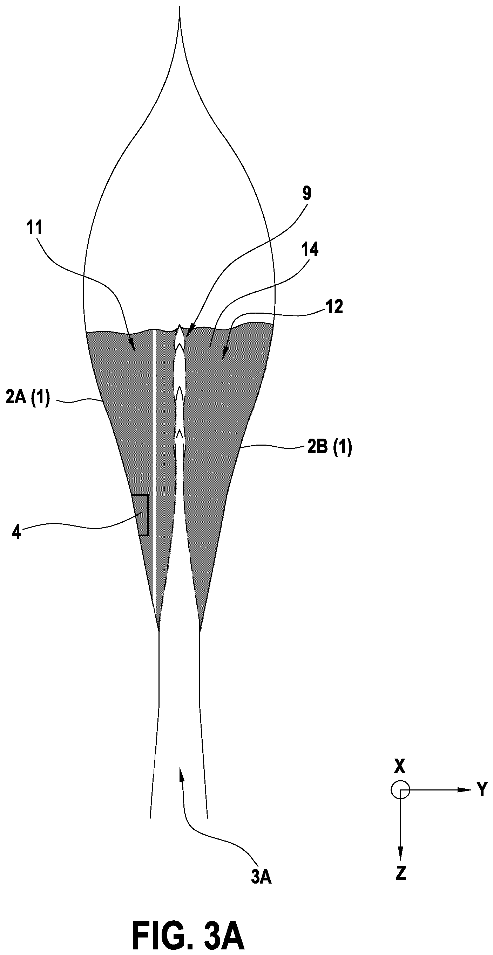

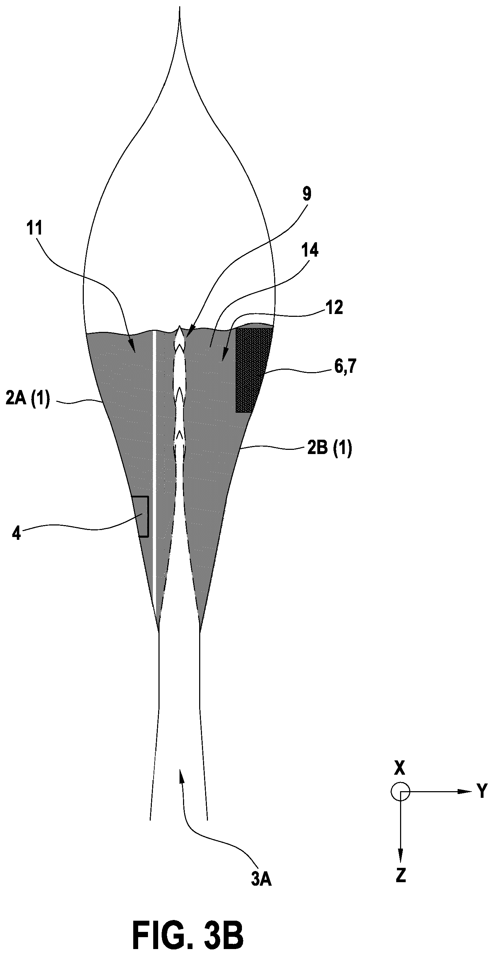

Comme représenté sur les figures 3A et 3B, la poche souple 1 peut ensuite être ensuite retournée de manière à baigner le dispositif d'analyse de liquides 4, et/ou le matériau absorbant 6, dans les liquides 14.

En particulier, les liquides 14 remplissent la première chambre 11 de manière à baigner le dispositif d'analyse de liquide 4, et/ou remplissent la deuxième chambre 12, de manière à baigner le matériau absorbant 6.

Lors du retournement de la poche souple 1, le clapet anti retour 9 fait obstacle à une fuite de liquides 14 de l'intérieur de la poche souple 1 vers l'extérieur de la poche souple 1.

L'extension du clapet anti retour 9 peut être prévue de manière adaptée au volume de liquides normalement recueillis dans la poche et donc reçus dans les chambres 11 et 12 lorsque la poche est retournée. En particulier, il peut être conçu de manière à assurer une séparation des liquides 14 dans la première chambre 11 et dans la deuxième chambre 12 sans connexion fluidique entre la première chambre 11 et la deuxième chambre 12, comme représenté sur les figures 3A et 3B.

Par exemple, dans le cas où les liquides recueillis sont les urines d'un utilisateur, la première chambre 11 et la deuxième chambre 12 peuvent être chacune prévue pour recueillir un volume supérieur à la moitié du volume moyen d'urine émis lors d'une miction d'urine.

On comprend toutefois que dans la position retournée de la poche souple 1, le niveau de liquides peut également dépasser l'extrémité du clapet anti retour 9.

Dans la première chambre 11, le dispositif d'analyse de liquides 4 baigne dans les liquides 14 afin de réaliser une mesure d'analyse de liquides. Afin de réaliser l'analyse des liquides, la durée du bain dans les liquides 14 doit correspondre à une durée d'exposition prédéterminée, qui est une plage de durées comprise entre une limite basse qui est une durée minimale de contact permettant aux réactions nécessaires à la mesure d'avoir lieu, et une limite haute qui est une durée maximale de contact au-delà de laquelle la mesure est dégradée, par exemple par dissolution de réactifs du dispositif d'analyse de liquides 4 dans les liquides 14. Cette durée d'exposition prédéterminée dépend de la nature du dispositif d'analyse de liquides 4 et des marqueurs utilisés, et est par exemple de l'ordre d'une dizaine à une centaine de secondes.

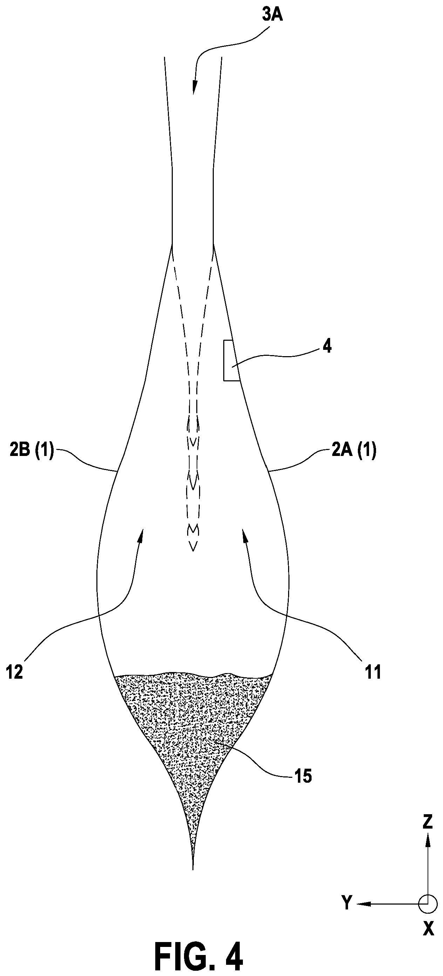

Après une durée correspondante à la durée d'exposition prédéterminée, la poche souple 1 peut de nouveau être retournée de manière à faire tomber les liquides 14 et le matériau absorbant 6 résiduel au fond de la poche souple 1, et le matériau absorbant 6 résiduel peut alors finir d'absorber les liquides 14 de manière à former un résidu 15 de liquides, comme représenté sur la figure 4.

Dans la deuxième chambre 12, le matériau absorbant 6 baignant dans les liquides 14 absorbe une partie des liquides 14. Dans le cas où le matériau absorbant 6 est prévu dans une enveloppe soluble 7, l'absorption des liquides par le matériau absorbant 6 commence après libération du matériau absorbant 6 suite à la dissolution de l'enveloppe soluble 7.

La quantité de matériau absorbant 6 est prévue pour pouvoir absorber la totalité des liquides 14 recueillis dans la poche souple 1. Par exemple, le matériau absorbant 6 peut contenir du polyacrylate de sodium, dans des quantités de l'ordre de l'unité à la dizaine de grammes.

Après une durée correspondante à la durée d'exposition prédéterminée, la poche souple 1 peut de nouveau être retournée de manière à faire tomber les liquides 14 et le matériau absorbant 6 résiduel au fond de la poche souple 1, et le matériau absorbant 6 résiduel peut alors finir d'absorber les liquides 14 de manière à former un agrégat 15 de liquides absorbés, comme représenté sur la figure 4.

Les résultats de mesures du dispositif d'analyse de liquides 4 peuvent alors être relevés, par lecture optique ou par transmission par une liaison sans fil vers un serveur. Ces résultats de mesures peuvent alors être utilisés par un personnel médical, qui pourra alors comparer ces valeurs mesurées à des valeurs nominales d'un patient en bonne santé puis réaliser un diagnostic. Ces résultats de mesures peuvent également être utilisés par un utilisateur autre qu'un personnel médical afin de détecter des taux anormaux de grandeurs d'intérêt, par exemple de taux de créatinine pour des sportifs ou de nitrites et de leucocytes pour des personnes susceptibles aux infections urinaires. Un utilisateur peut alors prendre contact avec un personnel médical apte à réaliser un diagnostic.

Dans le cas d'un dispositif d'analyse de liquides 4 nécessitant une lecture optique, celle-ci est de préférence réalisée de préférence à travers la poche souple 1, qui doit alors être au translucide ou transparente.

Le recueillement des liquides d'un utilisateur peut être réalisé de manière autonome par ce même utilisateur, ou avec l'assistance d'un autre utilisateur, par exemple un personnel médical.

La présente invention a été décrite dans le cas d'une poche souple 1 formée de l'assemblage d'une première paroi 2A et d'une deuxième paroi 2B. Toutefois, d'autres structures de poche souples peuvent être adoptées On comprend que dans ce cas, la première paroi et la deuxième paroi se distinguent en leur position relative au clapet anti retour 9, à la première chambre 11 et à la deuxième chambre 12.

La présente invention a été décrite dans le cas d'un agencement de protection 9 comprenant un clapet anti retour à feuilles. On comprend toutefois qu'un autre type de clapet anti retour peut être prévu, par exemple en fonction de la nature des liquides à recueillir.

La présente invention a été décrite dans le cas où le dispositif réactif comprend un dispositif d'analyse de liquide, et un matériau absorbant. On comprend que d'autres éléments réactifs à au moins un composant d'un liquide peuvent être prévus, et que chacun des éléments réactifs peut être prévu indépendamment des autres.

## Revendications

1. Dispositif de recueillement de liquides (100), comprenant :
une poche souple (1) comprenant des parois (2A, 2B) configurées pour ménager entre elles une ouverture d'introduction de liquides (3A),
un dispositif réactif comprenant au moins un élément réactif à au moins un composant d'un liquide (4, 6), et
un agencement de protection (9) configuré pour protéger le dispositif réactif vis-à-vis d'un contact avec un jet de liquide provenant de l'ouverture d'introduction de liquides (3A) ;
dans lequel le dispositif réactif comprend un dispositif d'analyse de liquides (4) et un matériau absorbant (6) ;
**caractérisé en ce que** l'agencement de protection (9) est configuré pour protéger le dispositif d'analyse de liquides (4) vis-à-vis d'un contact avec le matériau absorbant (6).

2. Dispositif de recueillement de liquides selon la revendication 1, dans lequel l'agencement de protection (9) est configuré pour autoriser le contact de liquides recueillis dans la poche souple (1) avec le matériau absorbant (6) et avec le dispositif d'analyse de liquides (4).

3. Dispositif de recueillement de liquides selon la revendication 1 ou 2, comprenant un clapet anti retour configuré pour faire obstacle à une circulation de liquides recueillis dans la poche souple (1) vers l'extérieur de la poche souple (1).

4. Dispositif de recueillement de liquides selon la revendication 3, dans lequel l'agencement de protection (9) comprend au moins une partie du clapet anti retour.

5. Dispositif de recueillement de liquides selon la revendication 3 ou 4, dans lequel le clapet anti retour comprend un clapet anti retour à feuilles.

6. Dispositif de recueillement de liquides selon l'une quelconque des revendications 1 à 5, dans lequel l'agencement de protection (9) comprend une portion de feuilles (8) souples qui délimite une première chambre (11) avec une première paroi (2A) de la poche souple (1) et qui délimite une deuxième chambre (12) avec une deuxième paroi (2B) de la poche souple (1).

7. Dispositif de recueillement de liquides selon la revendication 6, dans lequel le dispositif d'analyse de liquides (4) est situé dans la première chambre (11) et le matériau absorbant (6) est situé dans la deuxième chambre (12), le dispositif d'analyse de liquides (4) et le matériau absorbant (6) étant positionnés dans des zones comprises entre l'ouverture d'introduction de liquides (3A) et une extrémité de l'agencement de protection (9) opposée à l'ouverture d'introduction de liquides (3A).

8. Dispositif de recueillement de liquides selon la revendication 6 ou 7, dans lequel le dispositif d'analyse de liquides (4) est fixé à l'une de la première paroi (2A) et de la portion de feuilles (8) souples, et le matériau absorbant (6) est fixé à l'une de la deuxième paroi (2B) et de la portion de feuilles (8) souples.

9. Dispositif de recueillement de liquides selon l'une quelconque des revendications 1 à 8, dans lequel le matériau absorbant (6) est prévu dans une enveloppe soluble (7).

10. Dispositif de recueillement de liquides selon l'une quelconque des revendications 1 à 9, dans laquelle le matériau absorbant (6) comprend du polyacrylate de sodium.

11. Dispositif de recueillement de liquides selon l'une quelconque des revendications 1 à 10, dans laquelle le dispositif d'analyse de liquides (4) comprend une bandelette réactive de test (4B).

12. Dispositif de recueillement de liquides selon l'une quelconque des revendications 1 à 11, dans laquelle le dispositif d'analyse de liquides (4) comprend un laboratoire sur puce (4A).

13. Procédé de recueillement de liquides d'un ou par un utilisateur à l'intérieur d'une poche souple comprenant des parois configurées pour ménager entre elles une ouverture d'introduction de liquides, un dispositif réactif comprenant au moins un élément réactif à au moins un composant d'un liquide, le procédé comprenant les étapes suivantes :
recueillement dans la poche souple de liquides d'un ou par un utilisateur à travers l'ouverture d'introduction de liquides ; protection du dispositif réactif vis-à-vis d'un contact avec un jet de liquide provenant de l'ouverture d'introduction de liquides (3A) ;
le procédé étant **caractérisé en ce qu'**il comprend la protection d'un matériau absorbant du dispositif réactif vis-à-vis d'un contact avec un dispositif d'analyse de liquides du dispositif réactif.

14. Procédé de recueillement de liquides d'un ou par un utilisateur selon la revendication 13, comprenant les étapes suivantes : entrave d'une circulation de liquides recueillie dans la poche souple (1) vers l'extérieur de la poche souple (1) ; retournement de la poche souple (1) de manière à baigner le dispositif d'analyse de liquides (4) et le matériau absorbant (6) dans les liquides recueillis dans la poche souple (1) pendant une durée d'exposition prédéterminée.

15. Procédé de recueillement de liquides d'un utilisateur selon la revendication 14, comprenant le relevé d'une mesure du dispositif d'analyse de liquides (4) et l'absorption de liquides recueillie dans la poche souple (1) par le matériau absorbant (6).

## Patentansprüche

1. Vorrichtung (100) zum Auffangen von Flüssigkeiten , umfassend:
einen flexiblen Beutel (1) mit Wänden (2A, 2B), die so ausgebildet sind, dass sie zwischen sich eine Flüssigkeitseinlassöffnung (3A) bilden,
eine Reaktionsvorrichtung, die mindestens ein Element umfasst, das auf mindestens eine Komponente einer Flüssigkeit (4, 6) reagiert, und
eine Schutzanordnung (9), die so ausgebildet ist, dass sie die Reaktionsvorrichtung vor einem Kontakt mit einem Flüssigkeitsstrahl aus der Flüssigkeitseinlassöffnung (3A) schützt;
wobei die Reaktionsvorrichtung eine Flüssigkeitsanalysevorrichtung (4) und ein Absorptionsmaterial (6) umfasst;
**dadurch gekennzeichnet, dass** die Schutzanordnung (9) so ausgebildet ist, dass sie die Flüssigkeitsanalysevorrichtung (4) vor einem Kontakt mit dem Absorptionsmaterial (6) schützt.

2. Flüssigkeitsauffangvorrichtung nach Anspruch 1, wobei die Schutzanordnung (9) so ausgebildet ist, dass sie den Kontakt von in dem flexiblen Beutel (1) aufgefangenen Flüssigkeiten mit dem Absorptionsmaterial (6) und mit der Flüssigkeitsanalysevorrichtung (4) ermöglicht.

3. Flüssigkeitsauffangvorrichtung nach Anspruch 1 oder 2, umfassend ein Rückschlagventil, das so ausgebildet ist, dass es einen Fluss von in dem flexiblen Beutel (1) aufgefangenen Flüssigkeiten aus dem flexiblen Beutel (1) heraus verhindert.

4. Flüssigkeitsauffangvorrichtung nach Anspruch 3, wobei die Schutzanordnung (9) mindestens einen Teil des Rückschlagventils umfasst.

5. Flüssigkeitsauffangvorrichtung nach Anspruch 3 oder 4, wobei das Rückschlagventil ein Lamellenrückschlagventil umfasst.

6. Flüssigkeitsauffangvorrichtung nach einem der Ansprüche 1 bis 5, wobei die Schutzanordnung (9) einen Abschnitt aus flexiblen Folien (8) umfasst, der eine erste Kammer (11) mit einer ersten Wand (2A) des flexiblen Beutels (1) begrenzt und eine zweite Kammer (12) mit einer zweiten Wand (2B) des flexiblen Beutels (1) begrenzt.

7. Flüssigkeitsauffangvorrichtung nach Anspruch 6, wobei sich die Flüssigkeitsanalysevorrichtung (4) in der ersten Kammer (11) befindet und sich das Absorptionsmaterial (6) in der zweiten Kammer (12) befindet, wobei die Flüssigkeitsanalysevorrichtung (4) und das Absorptionsmaterial (6) in Bereichen zwischen der Flüssigkeitseinlassöffnung (3A) und einem der Flüssigkeitseinlassöffnung (3A) gegenüberliegenden Ende der Schutzanordnung (9) angeordnet sind.

8. Flüssigkeitsauffangvorrichtung nach Anspruch 6 oder 7, wobei die Flüssigkeitsanalysevorrichtung (4) an der ersten Wand (2A) oder dem flexiblen Folienabschnitt (8) befestigt ist und das Absorptionsmaterial (6) an der zweiten Wand (2B) oder dem flexiblen Folienabschnitt (8) befestigt ist.

9. Flüssigkeitsauffangvorrichtung nach einem der Ansprüche 1 bis 8, wobei das Absorptionsmaterial (6) in einer löslichen Hülle (7) vorgesehen ist.

10. Flüssigkeitsaufnahmevorrichtung nach einem der Ansprüche 1 bis 9, wobei das Absorptionsmaterial (6) Natriumpolyacrylat umfasst.

11. Flüssigkeitsauffangvorrichtung nach einem der Ansprüche 1 bis 10, wobei die Flüssigkeitsanalysevorrichtung (4) einen Teststreifen (4B) umfasst.

12. Flüssigkeitsauffangvorrichtung nach einem der Ansprüche 1 bis 11, wobei die Flüssigkeitsanalysevorrichtung (4) ein Lab-on-a-Chip (4A) umfasst.

13. Verfahren zum Auffangen von Flüssigkeiten durch einen Benutzer in einem flexiblen Beutel, der Wände aufweist, die so angeordnet sind, dass sie zwisch eine Flüssigkeitseinlassöffnung bilden, sowie eine Reaktionsvorrichtung, die mindestens ein Element umfasst, das auf mindestens eine Komponente einer Flüssigkeit reagiert, wobei das Verfahren die folgenden Schritte umfasst:
Aufnehmen von Flüssigkeiten durch einen Benutzer in den flexiblen Beutel durch die Flüssigkeitseinlassöffnung; Schützen der Reaktionsvorrichtung vor einem Kontakt mit einem Flüssigkeitsstrahl, der aus der Flüssigkeitseinlassöffnung (3A) austritt;
wobei das Verfahren **dadurch gekennzeichnet ist, dass** es den Schutz eines absorbierenden Materials der Reaktionsvorrichtung vor einem Kontakt mit einer Flüssigkeitsanalysevorrichtung der Reaktionsvorrichtung umfasst.

14. Verfahren zum Auffangen von Flüssigkeiten von einem oder durch einen Benutzer gemäß Anspruch 13, umfassend die folgenden Schritte: Verhinderung eines Fließens der in dem flexiblen Beutel (1) gesammelten Flüssigkeiten aus dem flexiblen Beutel (1) heraus; Umdrehen des flexiblen Beutels (1), so dass die Flüssigkeitsanalysevorrichtung (4) und das Absorptionsmaterial (6) während einer vorbestimmten Einwirkzeit in die im flexiblen Beutel (1) gesammelten Flüssigkeiten getaucht werden.

15. Verfahren zum Sammeln von Flüssigkeiten eines Benutzers gemäß Anspruch 14, umfassend das Ablesen eines Messwerts der Flüssigkeitsanalysevorrichtung (4) und das Absorbieren der in dem flexiblen Beutel (1) gesammelten Flüssigkeiten durch das Absorptionsmaterial (6).

## Claims

1. A device for collecting liquids (100), comprising:
a flexible pouch (1) comprising walls (2A, 2B) configured to form an opening therebetween for introducing liquids (3A),
a reactive device comprising at least one element that is reactive with at least one component of a liquid (4, 6), and
a protective arrangement (9) configured to protect the reactive device from contact with a jet of liquid coming from the opening for introducing liquids (3A);
wherein the reactive device comprises a device for analysing liquids (4) and an absorbent material (6);
**characterized in that** the protective arrangement (9) is configured to protect the device for analysing liquids (4) from contact with the absorbent material (6).

2. The device for collecting liquids according to claim 1, wherein the protective arrangement (9) is configured to allow the contact of liquids collected in the flexible pouch (1) with the absorbent material (6) and with the device for analysing liquids (4).

3. The device for collecting liquids according to claim 1 or 2, comprising a non-return valve configured to obstruct a flow of liquids collected in the flexible pouch (1) towards the outside of the flexible pouch (1).

4. The device for collecting liquids according to claim 3, wherein the protective arrangement (9) comprises at least one part of the non-return valve.

5. The device for collecting liquids according to claim 3 or 4, wherein the non-return valve comprises a plurality of sheets.

6. The device for collecting liquids according to any one of claims 1 to 5, wherein the protective arrangement (9) comprises a portion of flexible sheets (8) which delimits a first chamber (11) with a first wall (2A) of the flexible pouch (1) and which delimits a second chamber (12) with a second wall (2B) of the flexible pouch (1).

7. The device for collecting liquids according to claim 6, wherein the device for analysing liquids (4) is located in the first chamber (11) and the absorbent material (6) is located in the second chamber (12), the device for analysing liquids (4) and the absorbent material (6) being positioned in zones between the opening for introducing liquids (3A) and an end of the protective arrangement (9) opposite the opening for introducing liquids (3A).

8. The device for collecting liquids according to claim 6 or 7, wherein the device for analysing liquids (4) is secured to one of the first wall (2A) and the portion of flexible sheets (8), and the absorbent material (6) is secured to one of the second wall (2B) and the portion of flexible sheets (8).

9. The device for collecting liquids according to any one of claims 1 to 8, wherein the absorbent material (6) is provided in a soluble envelope (7).

10. The device for collecting liquids according to any one of claims 1 to 9, wherein the absorbent material (6) comprises sodium polyacrylate.

11. The device for collecting liquids according to any one of claims 1 to 10, wherein the device for analysing liquids (4) comprises a reactive test strip (4B).

12. The device for collecting liquids according to any one of claims 1 to 11, wherein the device for analysing liquids (4) comprises a lab-on-a-chip (4A).

13. A method for collecting liquids of a or by a user, inside a flexible pouch comprising walls configured to form an opening therebetween for introducing liquids, a reactive device comprising at least one element that is reactive to at least one component of a liquid, the method comprising the following steps:
collecting, in the flexible pouch, liquids of a or by a user through the opening for introducing liquids; protecting the reactive device from contact with a jet of liquid coming from the opening for introducing liquids (3A)the method being **characterized in that** it comprises protecting an absorbent material of the reactive device from contact with a device for analysing liquids of the reactive device.

14. The method for collecting liquids of a or by a user according to claim 13, comprising the following steps:
hindering a flow of liquids collected in the flexible pouch (1) towards the outside of the flexible pouch (1); turning over the flexible pouch (1) so as to bathe the device for analysing liquids (4) and the absorbent material (6) in the liquids collected in the flexible pouch (1) for a predetermined exposure duration.

15. The method for collecting liquids of a user according to claim 14, comprising the reading of a measurement of the device for analysing liquids (4) and the absorption of liquids collected in the flexible pouch (1) by the absorbent material (6).
